# EUROPÄISCHE PATENTSCHRIFT

(11) **EP 2 937 058 B1**
(45) Veröffentlichungstag und Bekanntmachung des Hinweises auf die Patenterteilung: **07.10.2020**
(21) Anmeldenummer: 15164682.5
(22) Anmeldetag: 22.04.2015
(51) Int. Cl.: A61C 1/08, A61C 9/00

(54) **KOPFGETRAGENE PLATTFORM ZUR INTEGRATION VON VIRTUALITÄT IN DIE REALITÄT**
HEAD MOUNTED PLATFORM FOR INTEGRATION OF VIRTUALITY INTO REALITY
PLATE-FORME PORTÉE SUR LA TÊTE DESTINÉ À L'INTÉGRATION DE RÉALITÉ AMPLIFIÉE

(30) Priorität: 24.04.2014 DE 102014207749
(43) Veröffentlichungstag der Anmeldung: 28.10.2015
(73) Patentinhaber: Ellerbrock, Christof, 55218 Ingelheim (DE)
(72) Erfinder: Ellerbrock, Christof, 55218 Ingelheim (DE)
(74) Vertreter: Gulde & Partner

(56) Entgegenhaltungen:
- EP-A1- 1 739 642
- WO-A1-01/05161
- WO-A2-03/034705
- WO-A2-2012/075155
- JP-A- 2001 211 403
- US-A1- 2011 205 242
- US-A1- 2013 172 731

## Beschreibung

### Technisches Gebiet

Die Erfindung betrifft eine Vorrichtung zur Gewinnung und Verarbeitung von 3D-Bildern.

Ein Beispiel für eine Vorrichtung zur Gewinnung und Verarbeitung von 3D-Bildern ist ein optische 3D Scanner.

### Technischer Hintergrund

Optische 3D Scanner können in Gehäusen verbaut sein, die entweder handgehalten oder stativgestützt sind. Es gibt auch solche Varianten die stationär in einem Standgehäuse verbaut sind.

Optisch 3D Scanner finden in vielen Bereichen gewerbliche Anwendung, beispielsweise in der Medizintechnik, insbesondere der Zahnmedizintechnik (Dentaltechnik).

Dentale 3D Scanner werden dabei zumindest zum Teil in die Mundhöhle eingeführt. In diesem "Mundteil" kann sich beispielsweis ein Spiegel oder ein Prisma zur Umlenkung der optischen Achse befinden. Bei manchen Systemen ist dieser Teil abnehmbar und sterilisierbar. Über dieses Bauteil wird bei vielen Modellen ein Streifenlichtmuster auf das zu scannende Objekt projektziert. Die Ablenkung der Streifen wird dann über das eingebaute Kamerasystem detektiert.

Technische 3D Scanner basieren zum Teil auf der oben genannten Streifenlichtprojektionstechnik. Oftmals kommt auch die Laserprojektionstechnik zum Einsatz, bei der ein Laserstrahl über das zu scannende Objekt bewegt wird und dessen Veränderung detektiert wird.

Im Bereich der virtuellen Realität kommen Videobrillen zum Einsatz. Es gibt auch Videobrillen, die eine eingebaute Kamera haben, um zwischen der virtuellen Realität und der Realität umzuschalten. Weiterhin ist bekannt, der Realität Informationen durch Projektionen auf transparente Scheiben, durch die der Nutzer die Realität sieht, zu überlagern. Diese Technologie findet beispielsweise in Head-up Displays von Flugzeugen oder Kraftfahrzeugen und in Google Glases Anwendung. Aus EP 1 739 642 A1 ist ein digitales Vergrößerungsglassystem bekannt, welches eine Vorrichtung aufweist, die eine Kopfmontage umfasst, an der zwei Kameras mit je einem zugehörigem Monitor zur Darstellung eines jeweiligen Bildes der jeweiligen Kamera so angeordnet sind, dass optischen Achsen der Monitore mit optischen Achsen der Augen eines Nutzers der Vorrichtung zusammenfallen oder zumindest parallel zu den optischen Achsen der Augen eines Nutzers ausrichtbar sind. Jede Kamera kann für sich eine automatische fokussierende, mit einer Zoomlinse ausgestattete CCD Kamera sein.

### Zusammenfassung der Erfindung

Erfindungsgemäß wird ein neues Herangehen an das 3D Scannen gewählt.

Dazu wird eine Vorrichtung nach Anspruch 1 zur Gewinnung und Verarbeitung von 3D-Bildern erfindungsgemäß vorgeschlagen.

Die Erfindung betrifft eine Vorrichtung zur Gewinnung und Verarbeitung von 3D-Bildern, umfassend:a) eine Kopfmontage (6),b) zwei Kameras (2) an der Kopfmontage (6) angeordnet und so miteinander synchronisiert, dass sie sich auf einen gemeinsamen Punkt (4) mit selber Blende und selben Zoom scharfstellen, wobei eine der Kameras (2) eine Führungskamera ist, wobei die Führungskamera durch einen Benutzer auswählbar ist und die Synchronisation leitet, und wobei die Kameras (2) für eine 10x Vergrößerung ausgelegt sind,c) ein Monitor (1) je Kamera (2) zugeordnet, zur Darstellung eines jeweiligen Bildes der jeweiligen Kamera (2), wobei die Monitore (1) so an der Kopfmontage (6) angeordnet, dass optische Achsen (5) der Monitore (1) mit optischen Achsen der Augen (7) eines Nutzers der Vorrichtung zusammenfallen oder zumindest parallel zu den optischen Achsen der Augen eines Benutzers ausrichtbar sind, undd) eine Datenverarbeitungseinheit (8) zur Datenverarbeitung von Kamerabildern, dadurch gekennzeichnet, dass e) die Vorrichtung einen Fußschalter (10) umfasst, wobei der Fußschalter (10) an der Datenverarbeitungseinheit (8) angeschlossen ist, und wobei der Fußschalter die Vergrößerung und / oder einen Fokus der Kameras (2) steuert.

Die erfindungsgemäße Vorrichtung ermöglicht ein 3D Scannen, welches durch das natürliche Sehen mit zwei Augen gesteuert wird. Die Kopfmontage der erfindungsgemäßen Vorrichtung wie eine Brille auf den Kopf gesetzt. Die zwei Kameras nehmen Bilder des zu scannenden Objektes auf. Das Bild der linken bzw. rechten Kamera wird auf dem zugehörigen Monitor, der vor dem jeweiligen Auge angeordnet ist, wiedergegeben.

Neu ist, dass zwei Kamerasysteme eingebaut sind die die Funktion des Auges imitieren. Die beiden Kamerasysteme sind miteinander synchronisiert, so dass sie sich auf einen gemeinsamen Punkt scharfstellen. Auch Blende und Zoom sind bei beiden Kameras synchronisiert.

Da die beiden Kameras Bilder der Realität aufnehmen, können digitale Informationen mit den Bildern der Realität überlagert werden. Weiterhin ist es dank des synchronisierten Zooms möglich, ein vergrößertes Bild der Realität darzustellen. Diese Funktion der Vergrößerung ermöglicht sehr vielfältige neue Einsatzbereiche. So kann die Vorrichtung auch als Mikroskopbrille genutzt werden, da die Kameras mindestens für eine 10x Vergrößerung ausgelegt sind.

In einer bevorzugten Ausführungsform der Vorrichtung umfasst die Datenverarbeitung ein Überlagern digitaler Informationen mit den Kamerabildern.

Die digitalen Informationen können 3D-Röntgenbilder eines Zahns umfassen. Dabei können Wurzelkanäle, Zahnwurzeln, Nerven und/oder Blutgefäße angezeigt werden.

Die digitalen Informationen können zumindest teilweise aus einer Implantatplanung übernommen werden, wobei dann die Datenverarbeitung ein Überlagern einer Anzeige eines virtuellen Implantats mit den Kamerabildern umfasst.

Die Anzeige des virtuellen Implantats kann eine Visualisierung einer Implantatachse in Verlängerung des virtuellen Implantats umfassen.

Eine Oberfläche einer neuen Füllung kann transparent eingeblendet werden.

Eine in ihrer Höhe von einer Sollhöhe abweichende Teilfläche der Oberfläche kann farblich kodiert angezeigt werden.

Die Vorrichtung kann mindestens ein Haptik-Feedback-Instrument umfassen und dafür vorgerichtet sein, mittels des Haptik-Feedback-Instruments ein virtuelles Modell zu konstruieren.

Die Vorrichtung kann dafür vorgerichtet sein, einen optischen 3D-Abdruck eines beschliffenen Zahnes aufzunehmen, und einem System für Computerunterstütze Modellierung und/oder Herstellung zur Verfügung zustellen.

Die Datenverarbeitung kann auf einem angeschlossenen Computer erfolgen. Dann kann auf dem Computer auch eine Speicherung der Kamerabilder erfolgen. Die Kamerabilder können alternativ oder zusätzlich auf auch einem oder mehreren weiteren Monitoren angezeigt werden.

Die Steuerung des Gesamtsystems kann über die Datenverarbeitungseinheit, die beispielsweise in Form eines angeschlossenen Computers, realisiert ist, erfolgen.

Dabei ist ein Fußschalter an den Computer angeschlossen, über den Fokus und/oder Vergrößerung gesteuert werden können.

Ein weiterer an den Computer angeschlossener Fußschalter kann zur Steuerung der Aufnahme (Speicherung) von Einzelbildern oder Bildsequenzen dienen.

Lagesensoren können von der Kopfmontage umfasst sein, um die Lage der Brille detektieren zu können.

Abhängig von dem Benutzer kann zwischen der rechten und linken Kamera als Führungskamera umgeschaltet werden.

Die zwei Kameras können in der optischen Achse des Auges des Nutzers individuell verstellbar angeordnet sein.

Zusätzlich oder alternativ kann die Position der beiden Monitore an den Nutzer angepasst werden.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen beispielhaft und schematisch:
- Figur 1: eine Ausführungsform der Erfindung,
- Figur 2: die Ausführungsform aus Figur 1 in einer Ansicht von oben,
- Figur 3: die Ansicht von oben sowie weitere Elemente der Ausführungsform,
- Figuren 4 -9: von mit Zahnbildern überlagerte 3D-Röntgenbilder,
- Figur 10: eine vergleichbare Ausführungsform, die nicht Gegenstand der Erfindung ist, und
- Figur 11: noch eine vergleichbare Ausführungsform, die nicht Gegenstand der Erfindung ist.

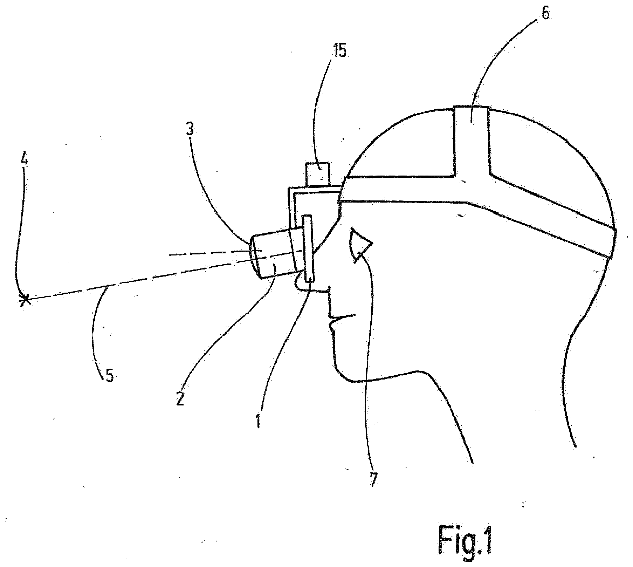

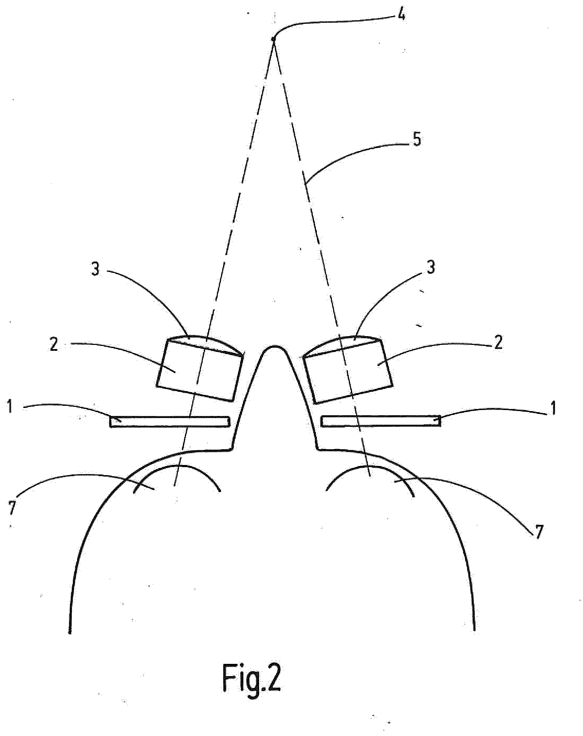

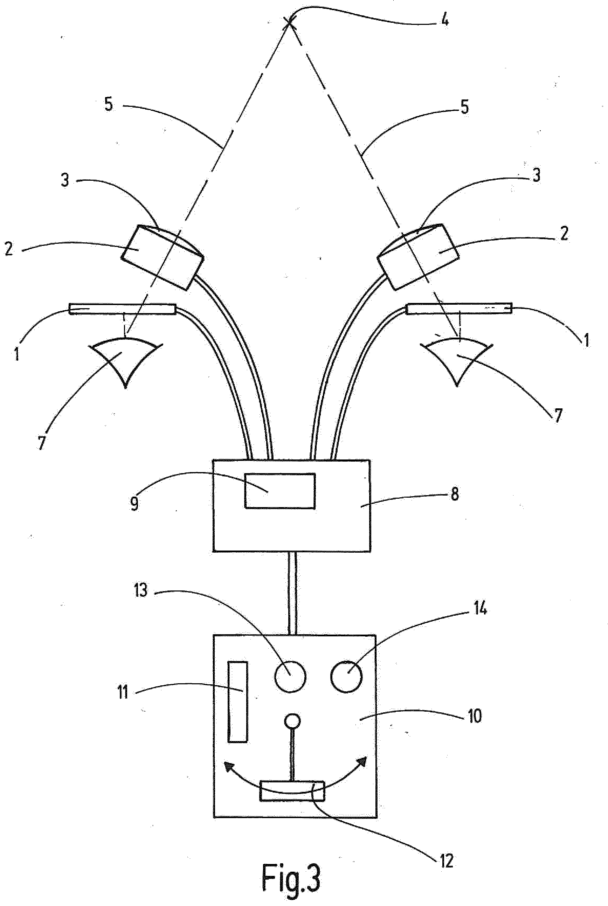

Figur 1 zeigt eine erste beispielhafte Ausführungsform der Erfindung in lateraler Ansicht. Figur 2 zeigt die Ausführungsform aus Figur 1 in einer Ansicht von oben. Figur 3 zeigt die Ansicht von oben sowie weitere Elemente der Ausführungsform.

Eine Vorrichtung zur Gewinnung und Verarbeitung von 3D-Bildern, beispielsweise in Form eines optischen 3D Scanners, umfasst eine Kopfmontage 6. Die Kopfmontage 6 ist eine Vorrichtung, mit der sich die Vorrichtung so auf dem Kopf eines Nutzers montiert werden kann, dass die Vorrichtung relativ zum Kopf fixiert ist. Die Kopfmontage 6 kann helm-, hut-, mützen- oder kappenartig ausgebildet sein. Es ist auch eine Kopfmontage 6 in Form eines Kopfhörers, der auf den Kopf oder die Ohren geklemmt wird, oder einer Brille möglich, die mittels auf dem Nasenbein und den Ohren ruhenden Elementen gehalten wird.

Die Kopfmontage 6 umfasst zwei Kameras 2 und zwei Displays (Monitore) 1. Beide Kameras 2und beide Displays 1 können an der "Kopfmontage" 6 befestigt sein. Die Kopfmontage 6 kann individuell an den Nutzer und insbesondere seine Augen 7 so anpassbar sein, dass optischen Achsen der Monitore 1 mit optischen Achsen der Augen 7 des Nutzers der Vorrichtung zusammenfallen. Beide Kamerasysteme 2 sind auf einen gemeinsamen Fokuspunkt 4 scharfgestellt. Die Entfernung des Fokuspunktes 4 kann mit der Vorrichtung variiert werden. Über die Zoomfunktion der Kamerasysteme 2 (digitaler Zoom) bzw. der eingesetzten Optik 3 (optischer Zoom) können die Kameras 2 das Objekt auch vergrößert darstellen. An einen Computer ist ein Fußschalter 10 angeschlossen, über den der Fokus 11 und die Vergrößerung 12 gesteuert werden können. Weiterhin befinden sich an ihm Fußtasten für die Foto 13 bzw. die Videofunktion 14. Da der Abstand der beiden Kameras 2 voneinander und deren Fokuspunkt 4 bekannt ist, lässt sich das Objekt dreidimensional vermessen bzw. erfassen. Außerdem können Lagesensoren 15 an die Kopfmontage montiert werden, um die Lage der Kopfmontage 6 zu detektieren. Diese Lagedaten können mit anderen Lagesensordaten verrechnet werden um eine relative Position zu ermitteln.

Ein beispielhafter gewerblicher Einsatzbereich der Erfindung ist in der Zahnmedizin. Beispielsweise kann die Erfindung als VR-Mikroskopbrille Verwendung finden. Hierbei wird mit den Kameras die Realität in Echtzeit aufgenommen und auf den Monitoren angezeigt. Durch die Zoom Funktion kann man das Bild vergrößert betrachten. Dabei sind Verstärkungen bis zu 10-fach möglich, ohne dass das Bild zu sehr verwackelt.

Durch die Möglichkeit der Videoaufnahme lassen sich Behandlungen aus Sicht des Behandlers dokumentieren. Weiterhin kann man die Funktion auch für Fortbildungen nutzen, indem eine Live-Übertragung erfolgt oder Videos aufgenommen werden können.

Durch die Überlagerung von Informationen in das Live-Bild kann man dem Behandler von außen Informationen einblenden. So kann beispielsweise eingeblendet werden, wo und in welcher Länge ein Schnitt gesetzt werden muss. Weiterhin kann ein Außenstehender sehen, sobald ein Skalpell nicht dem Schnitt entsprechend angesetzt wird, und dann korrigierend eingreifen. Ein Fehlschnitt wird so leichter verhinderbar.

Es ist auch möglich, dass ein Ausbilder seine VR-Mikroskopbrille auf die VR-Mikroskopbrillen seiner Auszubildenden oder Studenten schalten kann und so sieht, was der jeweilige Auszubildende sieht. So kann man als Ausbilder an vielen Orten gleichzeitig sein. Dabei ist es gleichgültig, wo sich der Ausbilder befindet, sofern ihm das Videosignal in Echtzeit oder annähernd in Echtzeit zur Verfügung steht.

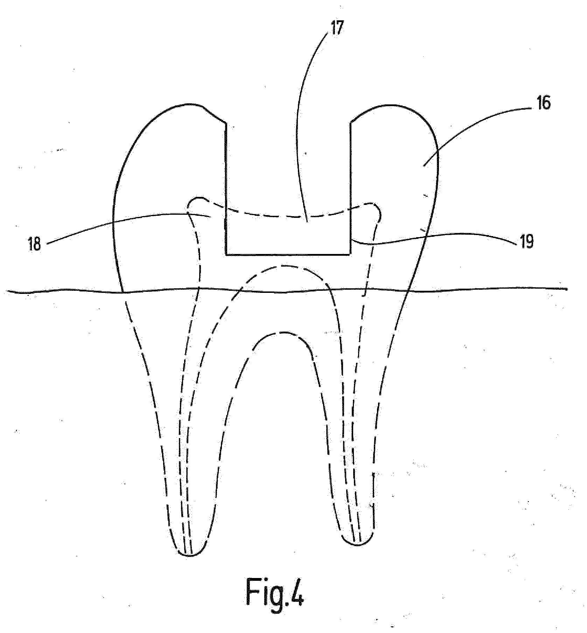

Figur 4 zeigt beispielhaft und schematisch ein mit einem 3D-Zahnbild überlagertes 3D-Röntgenbild im Rahmen einer Wurzelbehandlung. Der reale Zahn 16 ist mit dem 3D-Röntgenbild 17 überlagert. Wurzelkanäle 18 und Eingänge 19 der Wurzelkanäle 18, die sich in dem Zahn befinden werden, angezeigt. Das Bild aus 3D-Zahnbild überlagertes 3D-Röntgenbild kann gezoomt werden, so dass es einfacher ist die Eingänge 19 zu finden. Auch kann die Zahnwurzel in das Bild des realen Zahnes eingeblendet werden.

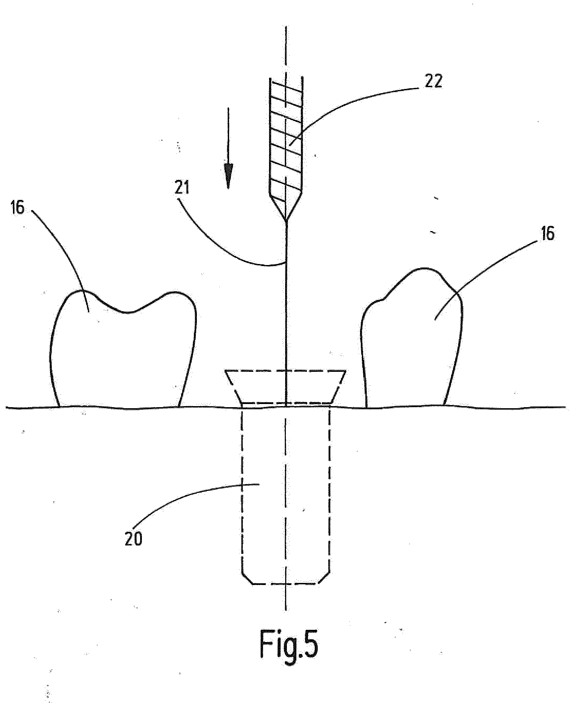

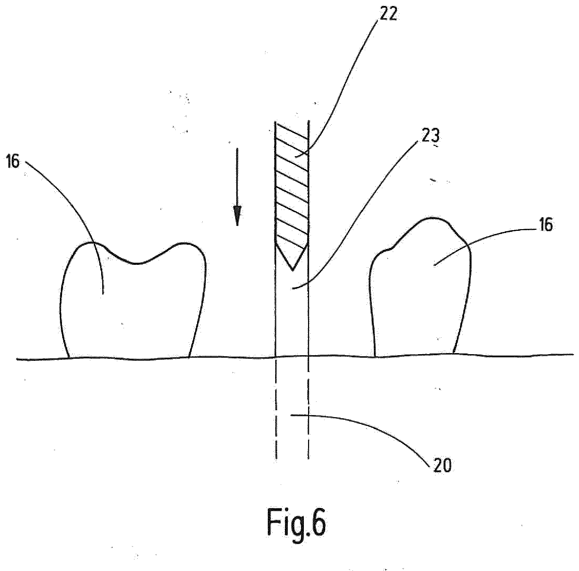

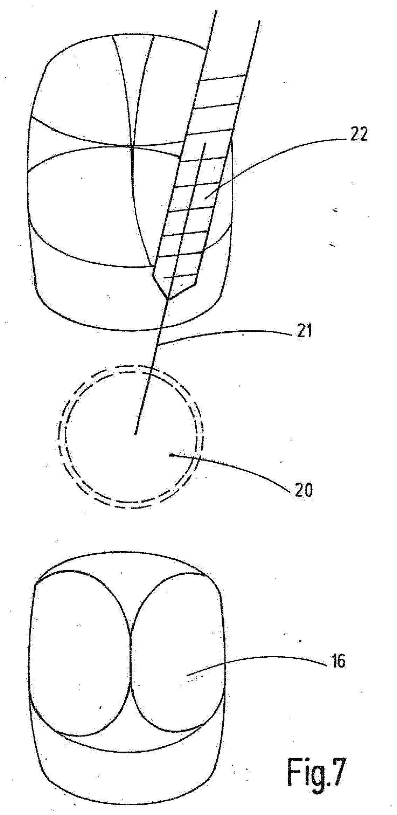

Figuren 5 bis 7 zeigen beispielhaft und schematisch mit 3D-Zahnbildern überlagerte 3D-Röntgenbilder im Rahmen einer Implantation. Bei der Überlagerung der 3D-Röntgenbilder mit der realen Situation wird die Implantatplanung 20 mit übernommen. Der Nutzer sieht also das Implantat 20 virtuell zwischen den realen Zähnen16. In Verlängerung des Implantates wird die Implantatachse 21 angezeigt. Der Nutzer kann dann einfach eine Implantatbohrerachse 22 in Übereinstimmung mit der Implantatachse 21 bringen, um die für das Implantat notwendige Bohrung in der richtigen Richtung und an der richtigen Position auszuführen. In einer Implantatplanungssoftware können auch die zu verwendeten Implantatbohrer in der entsprechenden räumlichen Ausrichtung schrittweise virtuell angezeigt werden, so dass man beim Bohren den realen Bohrer nur mit dem virtuell angezeigten Bohrer 23 überlagern muss. Für diese und andere Anwendungen muss aber die Lage der VR-Brille und die Position des Pateinten, als auch die Überlagerung Patient 3D-Röntgenbild bekannt sein. Dies kann mit der Lagesensorik 15 bewerkstelligt werden. Zusätzlich oder alternativ zu den Lagesensoren können auch Marker, beispielsweise Kugeln oder Körper mit optischen Mustern, an der Modellbasis befestigt sein, über die die Lage des Modellträgers berechnet werden kann.

Bei Operationen können gefährdete Bereiche wie z.B. Nerven oder große Gefäße eingeblendet werden. Die dafür notwendigen Informationen können von einer zuvor gemachten Diagnostik oder aus einem Modell stammen.

Beim Legen von Füllungen kann unter Verwendung eines 3D-Abdrucks des beschliffenen Zahnes an Hand von sog. Zahndatenbanken die Oberfläche einer neuen Füllung des beschliffenen Zahns berechnet werden. Diese Oberfläche kann transparent eingeblendet werden. Wird nun die Füllung modelliert können Flächen, die zu hoch modelliert sind, kodiert werden, aber auch solche die zu niedrig modelliert sind. Dies kann zudem bei gleichzeitiger Vergrößerung erfolgen, wobei zusätzlich unter Verwendung von 3D-Rötgenbildern sensible Bereiche im Zahn, beispielsweise der Nerv, angezeigt werden können.

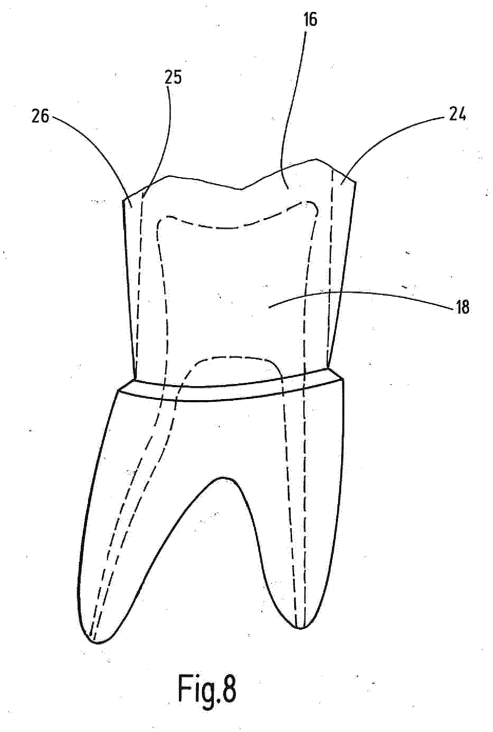

Figur 8 zeigt beispielhaft und schematisch ein mit einem 3D-Zahnbild überlagertes 3D-Röntgenbild im Rahmen des Beschleifens (Präparieren) eines Zahnes. Beim Beschleifen des Zahnes 16 für eine Krone/Brücke oder Teilkrone kann nach Eingabe des später verwendeten Materials der optimale Materialabtrag der Zahnsubstanz 24 angezeigt werden. Damit wird zum einen substanzschonend gearbeitet aber zum anderen wird genügend Material abgetragen um eine ausrechende materialabhängige Stabilität zu gewährleisten. Auch hier kann es zu einer farblichen Kodierung 26 kommen. Daten von 3D Röntgenbildern um den Zahnnerv 18 einzublenden können weiterhin genutzt werden. Es kann überprüft und optisch dargestellt werden, ob Zähne parallel ohne Unterschnitte 25 beschliffen wurden, um später eine Krone/Brücke auf die Zähne setzen zu können.

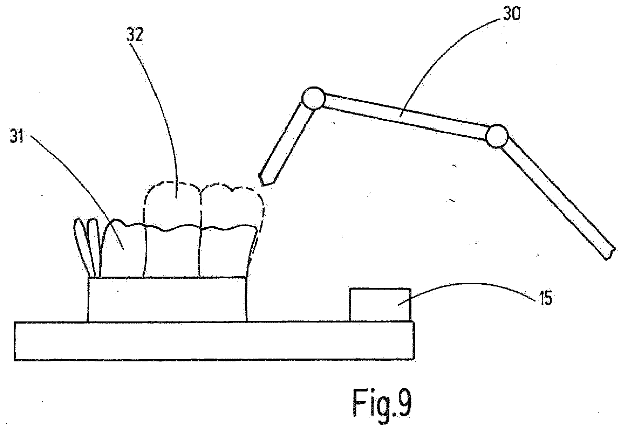

Figur 9 zeigt beispielhaft und schematisch ein mit einem 3D-Zahnbild überlagertes 3D-Röntgenbild im Rahmen der Zahntechnik. In Verbindung mit Haptik-Feedback-Instrumenten 30 ist es möglich, auf dem Modell des Patienten 31 Zahnersatz virtuell zu konstruieren und die Konstruktion 32 virtuell dem realen Modell zu überlagern. Durch Lagesensorik 15 kann der Zahntechniker das Modell in alle Richtungen drehen und wenden und bekommt immer die richtige Ansicht seiner virtuellen Modellierung 32 auf dem realen Modell 31 angezeigt. Zusätzlich oder alternativ zu den Lagesensoren können auch Marker, beispielsweise Kugeln oder Körper mit optischen Mustern, an der Modellbasis befestigt sein, über die die Lage des Modellträgers berechnet werden kann. Die Sensoren können dabei per drahtgebunden und/oder drahtlos, beispielsweise per Funk, mit einem Computer verbunden sein. Auch hier ist natürlich eine Vergrößerung der Ansicht möglich. Auch Zahndatenbanken können hier angezeigt werden, mit farblichen Kodierungen. Weitere Informationen über die Funktion, beispielsweise über die Bewegungen des Unterkiefers, können überlagert angezeigt werden. Beispielsweise können Bereiche des Zahnes, die bei Bewegungen des Unterkiefers stören könnten, angezeigt und auch wieder farbliche kodiert werden.

Es besteht auch die Möglichkeit, die VR-Mikroskopbrille auch als 3D-Kamera zur Aufnahme von beschliffenen Zähnen zu nutzen, um einen optischen Abdruck zu erstellen. Durch die mögliche Vergrößerung kann der Kronenrand viel genauer optisch detektiert und dargestellt werden. Die so aufgenommen Bilder bzw. Videosequenzen können in das stl-Datenformat umgerechnet werden. So können die Daten in jedem handelsüblichen CAD/CAM Programm verarbeitet werden.

Die Mikroskopbrille zeichnet sich in einer beispielhaften Ausführungsform technisch auch dadurch aus, dass der Augenabstand der beiden Displays variabel einstellbar ist..

Die zwei Kamerasysteme sind elektrisch miteinander synchronisiert.

Die optischen Achsen der Kamerasysteme sind in der Art zueinander ausgerichtet, dass sie horizontal entweder parallel sind oder so zueinander gewinkelt sind, dass sie einen gemeinsamen Fokuspunkt haben, der in einem Abstand von beispielsweise 15 cm bis Unendlich vor dem System liegt. In der Vertikalen ist die optische Achse entweder waagrecht oder bis zu einem Winkel von 80° nach unten geneigt.

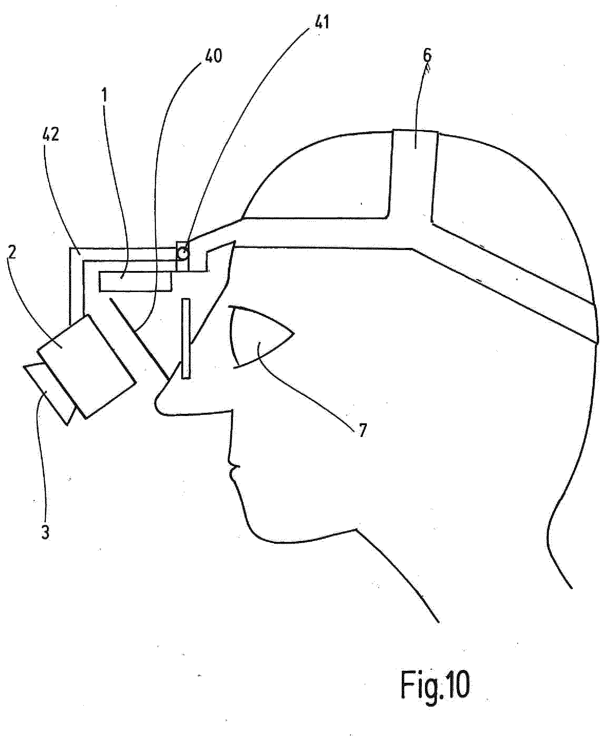

In einer weiteren vergleichbare Ausführungsform, die nicht Gegenstand der Erfindung ist, kann das System so aufgebaut werden, dass anstelle der beiden Displays auch halbtransparente optische Gläser eingesetzt werden, auf die die Kamerabilder projiziert werden können. Dies ist schematisch in Figur 10 gezeigt. Hier ist im Grundmodus keine Vergrößerung vorhanden, da der Anwender ohne technisches Sehsystem nur mit seinen eigenen Augen 7 durch die transparenten Gläser 40 sieht (ähnlich einer Sonnenbrille). Auf die transparenten Gläser 40 können nun virtuelle Informationen projiziert werden. Eine richtige Überlagerung von Informationen funktioniert jedoch nur wenn die projizierte Information die gleiche Größe wie das gesehene Objekt hat. Die Kopfmontage 6 umfasst einen um einen Drehpunkt 41 drehbaren Arm 42, an dem die Kamera 2 angeordnet ist.

Wird in den Mikroskopmodus geschaltet, d.h. die Information vergrößert dargestellt wird automatisch das halbtransparente optische Glas 40 komplett verdunkelt und das Bild des Kamerasystems in Verbindung mit den virtuellen Informationen angezeigt bzw. auf die nun komplett undurchsichtigen Gläser 40 projiziert. Die Kamerasystem 2 können bei dieser Brille nicht in der optische Achse des Auges montiert werden, sondern entweder seitlich oder über bzw. unter dem Auge. Ansonsten gelten für das Kamerasystem die gleichen Regeln wie vorher beschrieben.

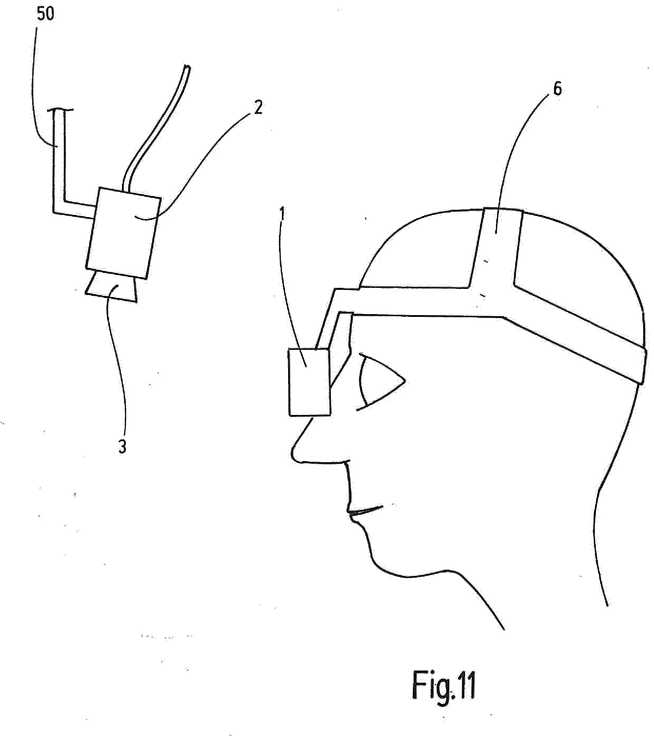

Eine noch weitere vergleichbare Ausführungsform, die nicht Gegenstand der Erfindung ist, ist schematisch in Figur 11 dargestellt. sieht die Trennung der optischen Systems vor. Hierbei wird das optische Aufnahmesystem 2 räumlich von dem Anzeigesystem 1 getrennt. Hierbei kann z.B. das optische Aufnahmesystem 2 an der OP Leuchte oder einem Stativ 50 gefestigt werden und die Informationen werden in dem Anzeigesystem 1 dargestellt. Hier kann die das Anzeigesystem derart modifiziert werden, dass die Augenbewegung und Blickrichtung über entsprechende Sensorik detektiert wird und sich das optische Aufnahmesystem entsprechend anpasst. Die Anpassung umfasst die Ausrichtung der optischen Achsen der beiden Kamerasysteme und die Lage des Fokuspunktes.

Dies sind nur einige beispielhafte Anwendungsmöglichkeiten in der Zahnmedizin bzw. Zahntechnik. Viele Anwendungen bzw. Vorgehensweisen sind in anderen Bereichen der Medizin aber auch in der Produktion ähnlich. Auch für Anwendung bei der Feuerwehr oder für militärische Anwendungen lässt sich eine erfindungsgemäße Vorrichtung als Teil eines geschlossen Helm realisieren. Ein geschlossener Helm bietet dem Träger mehr Sicherheit, wobei die Nutzung der Kameras weitere Funktionen ermöglicht. Eine nicht ausschöpfende Liste von Funktionsmöglichkeiten umfasst: Zoomen und vergrößertes Sehen, Darstellung von Gebäudeplänen und der eigenen Position innerhalb des Gebäudes, Darstellung von Kollegen, mögliche Anwendung von Farbfiltern und Infrarotdarstellung.

## Patentansprüche

1. Vorrichtung zur Gewinnung und Verarbeitung von 3D-Bildern, umfassend:
a) eine Kopfmontage (6),
b) zwei Kameras (2) an der Kopfmontage (6) angeordnet und so miteinander synchronisiert, dass sie sich auf einen gemeinsamen Punkt (4) mit selber Blende und selben Zoom scharfstellen, wobei eine der Kameras (2) eine Führungskamera ist, wobei die Führungskamera durch einen Benutzer auswählbar ist und die Synchronisation leitet, und wobei die Kameras (2) für eine 10x Vergrößerung ausgelegt sind,
c) ein Monitor (1) je Kamera (2) zugeordnet, zur Darstellung eines jeweiligen Bildes der jeweiligen Kamera (2), wobei die Monitore (1) so an der Kopfmontage (6) angeordnet, dass optische Achsen (5) der Monitore (1) mit optischen Achsen der Augen (7) eines Nutzers der Vorrichtung zusammenfallen oder zumindest parallel zu den optischen Achsen der Augen eines Benutzers ausrichtbar sind, und
d) eine Datenverarbeitungseinheit (8) zur Datenverarbeitung von Kamerabildern,
**dadurch gekennzeichnet, dass**
e) die Vorrichtung einen Fußschalter (10) umfasst, wobei der Fußschalter (10) an der Datenverarbeitungseinheit (8) angeschlossen ist, und wobei der Fußschalter die Vergrößerung und / oder einen Fokus der Kameras (2) steuert.

2. Vorrichtung nach Anspruch 1 wobei die Datenverarbeitung ein Überlagern digitaler Informationen mit den Kamerabildern umfasst.

3. Vorrichtung nach Anspruch 2 wobei die digitalen Informationen 3D-Röntgenbilder eines Zahns umfassen.

4. Vorrichtung nach Anspruch 3 wobei Wurzelkanäle (18), Zahnwurzeln, Nerven und/oder Blutgefäße angezeigt werden.

5. Vorrichtung nach einem der Ansprüche 2 - 4 wobei die digitalen Informationen zumindest teilweise aus einer Implantatplanung (20) übernommen werden, wobei die Datenverarbeitung ein Überlagern einer Anzeige eines virtuellen Implantats mit den Kamerabildern umfasst.

6. Vorrichtung nach Anspruch 5 wobei die Anzeige des virtuellen Implantats eine Visualisierung einer Implantatachse in Verlängerung des virtuellen Implantats umfasst.

7. Vorrichtung nach einem der Ansprüche 2 - 6 wobei eine Oberfläche einer neuen Füllung transparent eingeblendet wird.

8. Vorrichtung nach Anspruch 7 wobei eine in ihrer Höhe von einer Sollhöhe abweichende Teilfläche der Oberfläche farblich kodiert angezeigt wird.

9. Vorrichtung nach einem der Ansprüche 2 - 8 wobei die Vorrichtung mindestens ein Haptik-Feedback-Instrument (30) umfasst und dafür vorgerichtet ist, mittels des Haptik-Feedback-Instruments ein virtuelles Modell zu konstruieren.

10. Vorrichtung nach einem der vorangehenden Ansprüche wobei die Vorrichtung dafür vorgerichtet ist, einen optischen 3D-Abdruck eines beschliffenen Zahnes aufzunehmen, und einem System für Computerunterstütze Modellierung und/oder Herstellung zur Verfügung zustellen.

## Claims

1. A device for obtaining and processing 3D images, comprising:
a) a transom mounting (6),
b) two cameras (2) arranged on the transom mounting (6), and synchronized in such a way as to focus in on a common point (4) with the same aperture and same zoom, wherein the camera (2) is a main camera, wherein the main camera can be selected by a user and guides the synchronization, and wherein the cameras (2) are designed for 10x magnification,
c) a monitor (1) allocated to each camera (2) for displaying a respective image of the respective camera (2), wherein the monitors (1) are arranged on the transom mounting (6) in such a way that optical axes (5) of the monitor (1) coincide with optical axes of the eyes (7) of a user of the device, or at least can be aligned parallel to the optical axes of the eyes of a user, and
d) a data processing unit (8) for processing the data of camera images,
**characterized in that**
e) the device comprises a foot switch (10), wherein the foot switch (10) is hooked up to the data processing unit (8), and wherein the foot switch controls the magnification and/or a focus of the camera (2).

2. The device according to claim 1, wherein data processing involves overlapping digital information with the camera images.

3. The device according to claim 2, wherein the digital information comprises 3D X-ray images of a tooth.

4. The device according to claim 3, wherein root canals (18), tooth roots, nerves and/or blood vessels are displayed.

5. The device according to one of claims 2-4, wherein the digital information is derived at least partially from an implant plan (20), wherein data processing involves overlapping a display of a virtual implant with the camera images.

6. The device according to claim 5, wherein displaying the virtual implant involves visualizing an implant axis in an extension of the virtual implant.

7. The device according to one of claims 2-6, wherein a surface of a new filling is transparently overlaid.

8. The device according to claim 7, wherein a partial surface of the surface whose height deviates from a desired height is displayed color coded.

9. The device according to one of claims 2-8, wherein the device comprises at least one haptic feedback instrument (30), and is designed to construct a virtual model by means of the haptic feedback instrument.

10. The device according to one of the preceding claims, wherein the device is designed to record an optical 3D impression of a ground tooth, and make it available to a system for computer-assisted modeling and/or manufacture.

## Revendications

1. Dispositif d'acquisition et de traitement des images tridimensionnelles (3D), comprenant :
a) un montage porté sur la tête (6),
b) deux caméras (2) disposées sur le montage porté sur la tête (6) et synchronisées l'une avec l'autre de telle manière qu'elles se mettent au point sur un point commun (4) avec le même diaphragme et le même objectif à focale variable, sachant qu'une des caméras (2) est une caméra de guidage, sachant que la caméra de guidage peut être sélectionnée par un utilisateur et dirige la synchronisation et sachant que les caméras (2) sont conçues pour un agrandissement x 10,
c) un écran de contrôle (1) attribué à chaque caméra (2) pour la représentation d'une image respective de la caméra (2) respective, sachant que les écrans de contrôle (1) sont disposés sur le montage porté sur la tête (6) de telle manière que les axes optiques (5) des écrans de contrôle (1) coïncident avec les axes optiques des yeux (7) d'un utilisateur du dispositif ou peuvent être orientées au moins parallèlement aux axes optiques des yeux d'un utilisateur, et
d) une unité de traitement des données (8) pour le traitement des données d'images de caméra,
**caractérisé en ce que**
e) le dispositif comprend un interrupteur au pied (10), sachant que l'interrupteur au pied (10) est raccordé à l'unité de traitement des données (8) et sachant que l'interrupteur au pied commande l'agrandissement et/ou une mise au point focale des caméras (2).

2. Dispositif selon la revendication 1, sachant que le traitement des données comprend une superposition des informations numériques avec les images de caméra.

3. Dispositif selon la revendication 2, sachant que les informations numériques comprennent des radiographies tridimensionnelles d'une dent.

4. Dispositif selon la revendication 3, sachant que les canaux radiculaires (18), les racines dentaires, les nerfs et/ou vaisseaux sanguins sont affichés.

5. Dispositif selon l'une quelconque des revendications 2 - 4, sachant que les informations numériques sont prises en charge au moins en partie à partir d'une planification d'implant (20), sachant que le traitement des données comprend une superposition d'un affichage d'un implant virtuel avec les images de caméra.

6. Dispositif selon la revendication 5, sachant que l'affichage de l'implant virtuel comprend une visualisation d'un axe d'implant dans le prolongement de l'implant virtuel.

7. Dispositif selon l'une quelconque des revendications 2 - 6, sachant qu'une surface d'un nouveau remplissage est affichée en surimpression en transparence.

8. Dispositif selon la revendication 7, sachant qu'une partie de surface de la surface, s'écartant d'une hauteur théorique dans sa hauteur, est affichée en couleur de façon codée.

9. Dispositif selon l'une quelconque des revendications 2 - 8, sachant que le dispositif comprend au moins un instrument de retour d'informations haptique (30) et est réalisé à cet effet pour concevoir un modèle virtuel au moyen de l'instrument de retour d'informations haptique.

10. Dispositif selon l'une quelconque des revendications précédentes, sachant que le dispositif est réalisé à cet effet pour enregistrer une empreinte optique tridimensionnelle d'une dent polie et mettre à disposition un système pour la modélisation et/ou fabrication assistée par ordinateur.
